(19) Europäisches Patentamt
European Patent Office
Office européen des brevets

(11) **EP 3 582 751 B1**

(12) **EUROPÄISCHE PATENTSCHRIFT**

(45) Veröffentlichungstag und Bekanntmachung des Hinweises auf die Patenterteilung:
**06.09.2023   Patentblatt 2023/36**

(21) Anmeldenummer: **18707313.5**

(22) Anmeldetag: **16.02.2018**

(51) Internationale Patentklassifikation (IPC):
*A61K 9/00* (2006.01)     *A61K 47/32* (2006.01)
*A61K 47/36* (2006.01)     *A61K 47/38* (2006.01)
*A61K 31/122* (2006.01)

(52) Gemeinsame Patentklassifikation (CPC):
**A61K 9/0056; A61K 47/32; A61K 47/36; A61K 47/38**

(86) Internationale Anmeldenummer:
**PCT/EP2018/053920**

(87) Internationale Veröffentlichungsnummer:
**WO 2018/149983 (23.08.2018 Gazette 2018/34)**

(54) **STRUKTURIERTE ORODISPERGIERBARE FILME**

STRUCTURED ORODISPERSIBLE FILMS

FILMS ORODISPERSIBLES STRUCTURÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.02.2017   DE 102017103346**

(43) Veröffentlichungstag der Anmeldung:
**25.12.2019   Patentblatt 2019/52**

(73) Patentinhaber: **LTS Lohmann Therapie-Systeme AG**
**56626 Andernach (DE)**

(72) Erfinder:
• **STEINER, Denise**
**38114 Braunschweig (DE)**
• **KWADE, Arno**
**38176 Wendeburg (DE)**

(74) Vertreter: **Meissner Bolte Partnerschaft mbB Patentanwälte Rechtsanwälte**
**Postfach 86 06 24**
**81633 München (DE)**

(56) Entgegenhaltungen:
**WO-A1-2006/031209     WO-A1-2013/023775**
**WO-A1-2016/009001     JP-A- S6 336 767**

**Beschreibung**

Die vorliegende Erfindung betrifft strukturierte orodispergierbare Filme, die auf ihrer Unterseite eine geschlossene Oberfläche aufweisen und die auf ihrer Oberseite porös sind, so dass sie mit einem aktiven Wirkstoff beladen werden können. Die vorliegende Erfindung betrifft ebenfalls Verfahren zur Herstellung solcher orodispergierbarer Filme und die Verwendung von mit Wirkstoffen beladenen orodispergierbaren Filmen als Medikament.

Stand der Technik

Die perorale Gabe von Arzneistoffen stellt nach wie vor eine der häufigsten Arten der Verabreichung von Medikamenten dar. Traditionelle perorale Verabreichungsformen sind beispielsweise Tabletten oder Kapseln, die als Trägersysteme für die orale Verabreichung von Arzneistoffen verwendet werden.

Tabletten oder Kapseln werden in der Regel geschluckt, was bedingt, dass der Patient eine Flüssigkeit bereithält, mit der er diese Darreichungsform zu sich nehmen kann. Teilweise bestehen jedoch bei älteren Patienten oder Kindern Schluckbeschwerden, so dass diese die Einnahme von Tabletten oder Kapseln ablehnen bzw. eine Einnahme nur ungern erfolgt. Zudem ist es möglich, dass Tabletten und Kapseln vom Patienten längere Zeit im Mund behalten und dann ausgespuckt werden. Hieraus resultiert dann nicht selten eine schlechte Compliance, die sich nachteilig beim Heilungsfortschritt bzw. Therapieerfolg bemerkbar macht.

Zur Lösung der beschriebenen Probleme wurden in den letzten Jahren pharmazeutische Darreichungsformen entwickelt, wie insbesondere Granulate oder orale Filme, die ohne Flüssigkeitszufuhr eingenommen werden können und die rasch in der Mundhöhle zerfallen. Orale Filme zeichnen sich insbesondere dadurch aus, dass sie eine geringe Schichtdicke und eine große Oberfläche aufweisen und sich in kürzester Zeit (d.h. in den meisten Fällen 30 Sekunden oder weniger) im Mund auflösen. Sie können jederzeit und überall je nach Bedarf des Patienten auch diskret eingenommen werden. Dabei ist eine gleichzeitige Einnahme von Flüssigkeit nicht notwendig, da die in der Mundhöhle vorhandene Speichelflüssigkeit ausreicht, um den Film aufzulösen und den Wirkstoff freizusetzen.

Orale Filme, die pharmazeutische und nichtpharmazeutische Wirkstoffe enthalten, sowie Verfahren zu deren Herstellung werden unter anderem beschrieben in WO 2007/009800, WO 2007/009801 und WO 03/011259.

Die stetige Anpassung und Weiterentwicklung von Arzneiformen auf die individuellen Bedürfnisse der Patienten rückt seit einigen Jahren in der Pharmaindustrie und Forschung immer weiter in den Fokus. So soll es in Zukunft mit individualisierten Arzneimitteln möglich sein, Wirkstoffdosen direkt auf das Alter, das Geschlecht und die körperliche Statur des Patienten anzupassen. Um eine solche Anpassung kostengünstig verwirklichen zu können, werden neue Anforderungen an die bisher etablierten Arzneiformen gestellt.

So wurden beispielsweise wirkstofffreie Trägersubstanzen entwickelt, die in einem späteren Prozessschritt direkt für den Patienten in Apotheken oder Krankenhäusern mit individuellen Dosen mit einem oder mehreren pharmazeutischen Wirkstoffen beladen werden können. Eine dieser neuen und zukunftsweisenden Entwicklungen in den letzten Jahren war die Erforschung von orodispergierbaren Filmen. Diese haften bei Kontakt mit den Schleimhäuten direkt an und können somit von kleinen Kindern oder älteren Patienten nicht mehr ausgespuckt werden. Außerdem erleichtern sie die Einnahme von pharmazeutischen Wirkstoffen für Patienten mit Schluckbeschwerden. Die Wirkstoffaufnahme in den Körper kann über die Schleimhäute oder, beim Abschlucken der Stoffe, über den Darm erfolgen.

Ein Beispiel für einen orodispergierbaren Film, der für eine Anwendung im Rahmen eines individualisierten Arzneimittels geeignet sein soll, ist in der WO 2013/023775 A1 beschreiben. Die beschriebenen Filme weisen einen zweischichtigen Aufbau mit einer Basisschicht, die eine filmbildende Substanz enthält, und einer Oberschicht, die eine weitere filmbildende Substanz und einen Wirkstoff enthält, auf. Die Oberschicht kann dabei durch Aufdrucken in mehreren Schichten auf die Basisschicht aufgebracht werden, wodurch eine Beladung mit bis zu 1,12 mg pro 6 cm$^2$ Filmoberfläche realisiert werden konnte.

Ein wesentlicher Nachteil dieses Films, sowie anderer bisher bekannter orodispergierbaren Filme besteht jedoch darin, dass diese bisher nur kleine Wirkstoffmengen aufnehmen können. Dies hat zur Folge, dass diese Arzneiform bisher noch auf hochpotente Wirkstoffe begrenzt ist.

Vor diesem Hintergrund besteht ein Bedarf an einer Applikationsform für Wirkstoffe, die höhere Wirkstoffmengen aufnehmen kann, mit der aber andererseits die Vorteile von orodispergierbaren Filmen in Bezug auf die Einfachheit ihrer Anwendung realisiert werden. Die vorliegende Erfindung befasst sich mit diesem Bedarf.

Darstellung der Erfindung

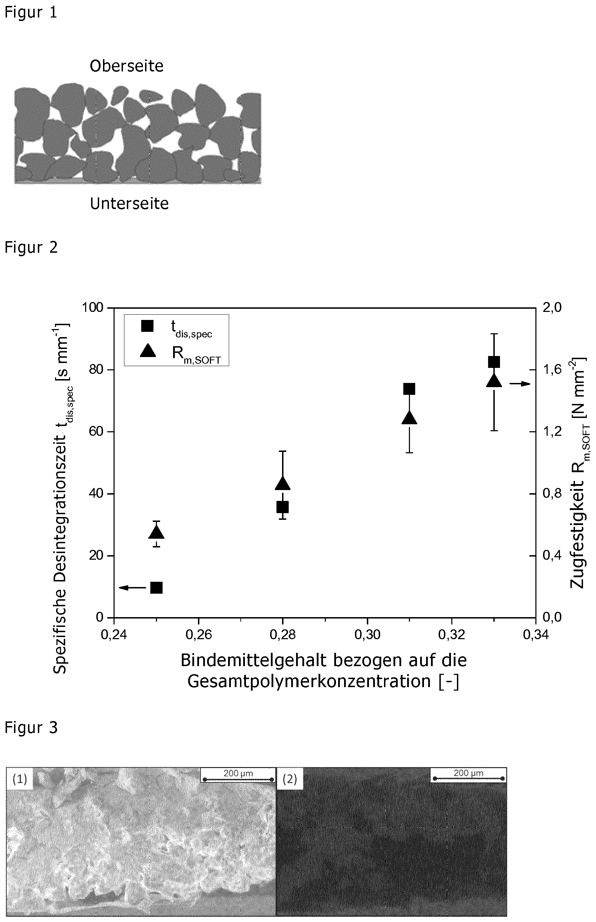

Gelöst wird das vorstehend geschilderte Problem durch orodispergierbare Filme, die über ihren Querschnitt eine inhomogene Porosität aufweisen. Insbesondere weisen die hier beschriebenen Filme auf einer ihrer Oberflächen (d.h. ihrer Oberseite) eine besonders hohe Porosität auf, was die Aufnahme eines Wirkstoffs, der von dieser Seite auf den Film aufgebracht wird, ermöglicht, während sie auf der dieser Seite abgewandten Seite (d.h. ihrer Unterseite) eine geschlossene Oberfläche aufweisen. Ein solcher Aufbau ist schematisch in Figur 1 gezeigt. Überraschend wurde im Rahmen der von den Erfindern durchgeführten Untersuchungen gefunden, dass sich solche Filme durch ein Verfahren, wie es in den Schritten i) bis iii) von Anspruch 1 angegeben ist, in einfacher Weise herstellen lassen. Die so hergestellten Filme können anschließend mit einem pharmazeutischen Wirkstoff beladen werden.

Ein erster Aspekt der vorliegenden Erfindung betrifft demzufolge ein Verfahren zur Herstellung eines orodispergierbaren Films, das die Schritte

i) Bilden einer Suspension aus einem pharmazeutisch akzeptablen Lösungsmittel, einem pharmazeutisch akzeptablen Matrixmaterial und einem pharmazeutisch akzeptablen Bindemittel, wobei das Lösungsmittel so ausgewählt ist, dass sich das pharmazeutisch akzeptable Matrixmaterial darin im Wesentlichen nicht löst, während das pharmazeutisch akzeptable Bindemittel im Lösungsmittel gelöst wird,
ii) Gießen des Dispersion auf einen neutralen Träger unter Bildung eines Nassfilms, und
iii) Trocknen des Nassfilms unter Erhalt eines porösen Trockenfilms, und weiterhin einen Schritt
iv) des Aufbringens einer Suspension oder Lösung eines pharmazeutischen Wirkstoffs in einem pharmazeutisch akzeptablen Lösungsmittel auf den aus Schritt iii) erhaltenen Trockenfilm und Trocknen des Films unterzogen wird, wobei ein orodispergierbarer Trockenfilm erhalten wird, dessen Poren zumindest anteilsmäßig mit dem pharmazeutischen Wirkstoff gefüllt sind, aufweist, umfasst.

Wie hier verwendet, bezeichnet der Ausdruck "orodispergierbarer Film" einen dünnen Film oder ein dünnes Blatt in jeder Form, einschließlich rechteckiger quadratischer oder anderer gewünschte Formen, die, wenn sie zum Beispiel durch Kontakt mit der Mundschleimhaut des Patienten oder bei oraler Gabe angefeuchtet werden, indem sie zum Beispiel auf die Zunge gelegt werden oder sublingual verabreicht werden, desintegrieren. Die Dicke und Größe der orodispergierbaren Filme, wie sie hier beschrieben werden, kann an die Mundhöhle des Benutzers ebenso wie auf die gewünschte Auflösungszeit abgestimmt werden.

Die Angabe, dass sich das pharmazeutisch akzeptabel Matrixmaterial in dem Lösungsmittel "im Wesentlichen nicht lösen" soll, ist so aufzufassen, dass die Löslichkeit des pharmazeutisch akzeptablen Matrixmaterials in dem Lösungsmittel nicht mehr als 1 g/L beträgt, und vorzugsweise nicht mehr als 0,5 g/L und besonders bevorzugt nicht mehr als 0,1 g/L, bestimmt jeweils bei Umgebungstemperatur (23°C), betragen sollte. Für die Löslichkeit ist dabei zu beachten, dass nicht mehr als die angegebene Menge des pharmazeutisch akzeptablen Matrixmaterials zu dem Zeitpunkt in dem Lösungsmittel gelöst sein sollte, an dem die Suspension auf den neutralen Träger aufgebracht wird. Das pharmazeutisch akzeptable Matrixmaterial kann demzufolge auch ein Material sein, das sich nur sehr langsam in dem Lösungsmittel löst, wenn das Aufbringen auf den neutralen Träger zu einem Zeitpunkt erfolgt, an dem sich maximal die angegebene Menge an pharmazeutisch akzeptablem Matrixmaterial in dem Lösungsmittel gelöst hat.

Bevorzugt bezeichnen die vorstehenden Löslichkeitsangaben jedoch die Löslichkeit des pharmazeutisch akzeptablen Matrixmaterials im Lösungsmittel unter Gleichgewichtsbedingungen.

Mit der Vorgabe, dass sich das pharmazeutisch akzeptable Bindemittel im Lösungsmittel "löst", soll im Rahmen der vorliegenden Erfindung verstanden werden, dass das pharmazeutisch akzeptable Bindemittel in dem Lösungsmittel eine Löslichkeit von mindestens 10 g/l, bevorzugt mindestens 50 g/l und meist bevorzugt mindestens 100 g/l aufweisen soll.

Mit einem "neutralen Träger" wird im Rahmen der vorliegenden Erfindung ein Träger bezeichnet, der keinerlei Wechselwirkung mit der aufgebrachten Suspension eingeht. Der neutrale Träger ist vorzugsweise so beschaffen, dass der nach dem Trocknen des Nassfilms erhaltene Trockenfilm leicht von dem neutralen Träger abgelöst werden kann, ohne dass es zum Reißen oder Brechen des Films kommt.

Im Rahmen des in Schritt iii) geschilderten Trocknens des Nassfilms unter Erhalt eines Trockenfilms ist es grundsätzlich möglich, die Wärme von unten oder von oben zuzuführen. Mit "von unten" oder "von oben" ist gemeint, dass die Wärmequelle oberhalb oder unterhalb des Films positioniert ist.

In einer besonders bevorzugten Ausführungsform wird die Wärme in Schritt iii) von unten, zweckmäßig über den neutralen Träger, zugeführt. Dies kann einerseits dadurch erfolgen, dass die Wärmequelle unterhalb des neutralen Trägers positioniert ist, es ist aber auch möglich, dass unterhalb des neutralen Trägers ein Wärme besonders gut absorbierendes Material positioniert ist und dass die Wärme von der originären Wärmequelle auf dieses Material übertragen wird. Ein geeignetes Material für eine solche Wärmeübertragung ist beispielsweise ein Metall oder Keramiksubstrat, das direkt unterhalb des neutralen Trägers positioniert wird.

Wie vorstehend erwähnt, besteht ein wesentlicher Vorteil des durch das vorstehend geschilderte Verfahren gemäß den Schritten i) bis iii) erhältlichen orodispergierbaren Films darin, dass er nachträglich mit einem Wirkstoff "beladen" werden kann. D.h., ein nachträglich z.B. als Lösung oder Suspension auf den porösen orodispergierbaren Film aufgebrachter Wirkstoff wird in die Poren des orodispergierbaren Films eingelagert, so dass sich ein einheitlicher, mit dem Wirkstoff beladener Film bildet. Dieser Film weist zwar in seinem Durchschnitt keine einheitliche Wirkstoffkonzentration auf, da die Wirkstoffkonzentration auf der Oberseite, an denen das Filmmaterial stärker porös ist, naturgegeben höher ist als auf Unterseite, an dem der Film solider ist, es ergibt sich aber keine klassische Zweischichtenstruktur, bei der der Wirkstoff in einer Schicht nur auf der Oberfläche des orodispergierbaren Films vorliegt.

Entsprechend umfasst das vorstehend geschilderte Verfahren nach dem Schritt iii) einen Schritt, bei dem der dort erhaltene Trockenfilm mindestens einen Schritt iv) des Aufbringens einer Suspension oder Lösung eines pharmazeutischen Wirkstoffs in einem pharmazeutisch akzeptablen Lösungsmittel auf den Trockenfilm und Trocknen des Films unterzogen wird. Durch dieses Vorgehen wird ein orodispergierbarer Trockenfilm erhalten, dessen Poren zumindest anteilsmäßig mit dem pharmazeutischen Wirkstoff gefüllt sind.

In Einzelfällen kann es sinnvoll sein, den so erhaltenen Film mit einer Schutzschicht zu versehen. In einer Ausgestaltungsform des vorstehend geschilderten Verfahrens kann es daher zweckmäßig sein, wenn der aus iv) erhaltene Film einem Schritt v) des Aufbringens einer Suspension oder Lösung eines pharmazeutisch akzeptablen Bindemittels in einem pharmazeutisch akzeptablen Lösungsmittel auf dem aus Schritt iv) erhaltenen Trockenfilm unterzogen wird. Durch dieses Vorgehen wird ein orodispergierbarer Trockenfilm erhalten, dessen Poren zumindest anteilsmäßig mit dem pharmazeutischen Wirkstoff gefüllt sind und der mit einer Schutzschicht aus dem Bindemittel überzogen ist.

Bei dem im Vorstehenden erwähnten pharmazeutisch akzeptablen Lösungsmittel kann es sich grundsätzlich um jedes im Stand der Technik bekannte pharmazeutisch akzeptable Lösungsmittel handeln. Denkbar sind als Lösungsmittel insbesondere Alkohole, umfassend Mono- und Polyalkohole (zum Beispiel Glykole), Ester, Ketone und Mischungen davon. Wasser ist als Bestandteil des pharmazeutisch akzeptablen Lösungsmittels ebenfalls möglich, dessen Anteil im Lösungsmittel sollte aber auf ein Minimum begrenzt sein, da sich der orodispergierbare Film bei Kontakt mit Flüssigkeit im Mund auflösen soll. Die Verwendung von substantiellen Anteilen an Wasser in Kombination mit dem Matrixmaterial kann daher die Ausbildung der porösen Oberflächenstruktur des orodispergierbaren Films beeinträchtigen, so dass dessen Anteil auf maximal 20 Vol.-%, vorzugsweise maximal 10 Vol.-% und besonders bevorzug maximal 5 Vol.-%, bezogen auf die Gesamtmenge an Lösungsmittel, begrenzt sein sollte.

Als Alkohole geeignet sind insbesondere kurzkettige Alkohole mit 1 bis 6 Kohlenstoffatomen, insbesondere 2, 3 oder 4 Kohlenstoffatomen, wie Methanol, Ethanol und Propanol, einschließlich 1-Propanol und 2-Propanol. Geeignete Polyalkohole beinhalten unter anderem Ethylenglykol und Propylenglykol. Ein besonders bevorzugter Alkohol als pharmazeutisch akzeptables Lösungsmittel ist Ethanol. Ein geeignetes Esterlösungsmittel ist beispielsweise Ethylacetat. Ein geeignetes pharmazeutisch akzeptables Ketonlösungsmittel ist Aceton. Es können auch Gemische der vorgenannten Lösungsmittel verwendet werden.

Als geeignete pharmazeutisch akzeptable Matrixmaterialien kommen sowohl synthetische Polymere als auch natürliche Polymere, wie insbesondere Polysaccharide in modifizierter oder nicht modifizierter Form, in Betracht. Als bevorzugte synthetische Polymere können

nen beispielsweise Polyvinylalkohol, Polyethylenglykol, vernetztes Polyvinylpyrrolidon und dessen Copolymere genannt werden. Besonders geeignete Polysaccharide umfassen Cellulose und Derivate davon, wie insbesondere Hydroxyalkylmethylcellulose und bevorzugt Hydroxyethylmethylcellulose und/oder Hydroxypropylmethylcellulose, Stärke, Stärkederivate, modifizierte Stärke, wie Maltodextrin, Di- und Oligosaccharide (mit 2 bis 10 Zuckereinheiten) und Glykomannane. Ein weiteres geeignetes, pharmazeutisch akzeptables Matrixmaterial ist Alginat.

Als besonders geeignetes pharmazeutisch akzeptables Matrixmaterial ist Hydroxypropylmethylcellulose, insbesondere eine Hydroxypropylmethylcellulose mit einem Hydroxypropyl-Anteil von 5 % bis 15 %, einem Methylanteil von 25 % bis 35 % und einer Viskosität von etwa 6 MPa·s als 2 %-ige wässrige Lösung in 20°C zu nennen. Die Mengen von Hydroxypropyl und Methyl beziehen sich auf die relativen Mengen im Verhältnis zu den Monomereinheiten des Polymers. Daher enthalten in einem Polymer, das 10% Hydroxypropyl enthält, 10% oder 1 von 10 Monomereinheiten des Polymers einen Hydroxypropylsubstituenten.

Spezifische Beispiele von geeigneten pharmazeutisch akzeptablen Matrixmaterialien sind verschiedene kommerziell erhältliche Hydroxypropylmethylcelluloseprodukte, die unter den Handelsnamen Pharmacoat 606 (Hydroxypropylmethylcellulose, Viskosität 6 MPa.s, Shin-Etsu Chemical Co. Ltd., Japan), Methocell E3 und Methocell E5 (Hydroxypropylmethylcellulose, Viskosität 3 MPa.s bzw. 5 MPa.s, Dow Chemical Company, USA) erhältlich sind. Ein ebenfalls besonders geeignetes Matrixmaterial ist Laktose.

Das pharmazeutisch akzeptable Matrixmaterial kann auch aus einem Gemisch mehrerer der vorstehend erwähnten spezifischen Matrixmaterialien bestehen, z.B. aus einem Gemisch von Hydroxypropylmethylcellulose und Laktose.

Der Partikelgröße des pharmazeutischen Matrixmaterials kommt im Rahmen der vorliegenden Erfindung eine Bedeutung zu, da sich die Partikelgröße in einem gewissen Umfang auf die Art und Größe der Poren auswirkt, die sich infolge des Trocknens des Nassfilms ausbilden. Als geeignete mittlere Teilchengröße für das pharmazeutisch akzeptable Matrixmaterial kann ein Bereich von 10 bis 200 μm, insbesondere 50 bis 150 μm, bevorzugt 70 bis 130 μm und besonders bevorzugt 80 bis 120 μm angegeben werden.

Die Teilchengröße bezeichnet in diesem Fall die volumetrisch gewichtete Teilchengröße und wird mittels der Laserbeugungsmethode bestimmt.

In Bezug auf das pharmazeutisch akzeptable Bindemittel können im Rahmen der vorliegenden Erfindung synthetische Polymere, zum Beispiel in Form von Polyvinylpyrrolidon, oder Polysaccharide, bevorzugt in Form von Cellulosederivaten und insbesondere Hydroxypropylcellulose, angegeben werden. Als bevorzugtes Bindemittel ist Hydroxypropylcellulose zu nennen, die unter anderem als HPC von Sigma Aldrich erhältlich ist.

Besonders bevorzugte Matrixmaterial-/ Bindemittelkombination sind zum Beispiel Hydroxypropylmethylcellulose (Matrix)/ Hydroxypropylcellulose (Binder) oder Laktose (Matrix)/Hydroxypropylcellulose (Binder).

Der Menge des pharmazeutisch akzeptablen Bindemittels in Bezug auf die Gesamtmenge an pharmazeutisch akzeptablem Matrixmaterial und Bindemittel kommt im Rahmen der vorliegenden Erfindung eine gewisse Bedeutung zu. So hat sich gezeigt, dass für sehr geringe Bindemittelgehalte keine geschlossene Unterseite des Films erhalten werden konnte, was zu einer wesentlich verminderten Festigkeit des Films führte. Bei zu hohen Bindemittelgehalten bildete sich hingegen ein deutlich dichterer Film, der eine geringere Porosität aufweist, was die Aufnahmefähigkeit des Films für einen nachträglich aufgebrachten pharmazeutischen Wirkstoff wesentlich vermindert. Als geeigneter Anteil von pharmazeutisch akzeptablem Bindemittel an der Gesamtmenge an pharmazeutisch akzeptablem Matrixmaterial und Bindemittel kann ein Bereich von 0,1 bis 0,5, insbesondere 0,2 bis 0,4, bevorzugt 0,25 bis 0,33 und besonders bevorzugt 0,27 bis 0,31, angegeben werden.

Auch der Lösungsmittelmenge kommt im Rahmen des vorstehend geschilderten Verfahrens eine gewisse Bedeutung zu. So wurde gefunden, dass die Suspension bei einer zu geringen die Lösungsmittelmenge eine zu hohe Viskosität aufwies, was zu uneinheitlich dicken Filmen und einer erhöhten Gefahr von Verstopfungen der zum Auftragen der Suspension verwendeten Düse führte. Andererseits ist auch ein zu hoher Anteil an Lösungsmittel ungünstig, da dieses im Rahmen des Trocknens aus dem Filmprodukt entfernt werden muss, was sich energetisch ungünstig bemerkbar macht. Als geeigneter Anteil des Lösungsmittels, bezogen auf das Gesamtgewicht der Suspension, kann ein Anteil im Bereich von 0,4 bis 0,9, insbesondere 0,68 bis 0,8, bevorzugt 0,7 bis 0,76 und besonders bevorzugt von 0,71 bis 0,75, angegeben werden.

Bei dem pharmazeutischen Wirkstoff kann es sich grundsätzlich um jeden oral verabreichbaren pharmazeutischen Wirkstoff handeln. Insbesondere ist der pharmazeutische Wirkstoff für orale Applikationen geeignet. Beispiele von geeigneten pharmazeutischen Wirkstoffen sind antiallergische Mittel, antiarhythmische Mittel, Antibiotika, antidiabetische Mittel, anti-epileptische Mittel, Antihistaminika, Antitussiva, cardiotronische Mittel, Diuretika, blutdrucksenkende Mittel, Betäubungsmittel, Nervenmuskelblocker und Sexualhormone, wie Vasopressoren. Spezifische Beispiele sind Acetaminophen, Adrenalin, Alprazolam, Amlodipin Besilat, Anastrozol, Apomorphin, Aripiprazol, Atorvastatin Calcium, Baclofen, Benzocain, Benzocain/Menthol, Benzydamin, Buprenorphin, Buprenorphin/Naloxon, Buprenorphin/Naloxon/Cetirizin, Cetirizin, insbesondere in Form von Cetirizine HCl, Cannabinoide, Chlorpheniramin, Clomipramin, DBP-166, Dexamethason, Dextromethorphan, Dextromethorphan/Phenylephrin, Diclofenac, Diphenhydramin, insbesondere in Form von Diphenhydramin Hydrochlorid, Diphenhydramin/Phenylephrin, Donepezil, insbesondere in Form von Donepezil Hydrochlorid, Dronabinol, Epinephrin, Escitalopram, Famotidin, Fentanyl, Glimepirid, GLP-1 Peptiden, Granisetron, Insulin, Insulin Nanopartikel, Insulin/GLP-1 Nanopartikel, INT-0020, INT-0022, INT-0023, INT-0025, INT-0030, INT-0036, INT-0031/2012, Ketoprofen, Ketotifen Fumarat, Koffein, Levocetirizin, Loperamid, Loratadin, Medizin Hydrochlorid, Methylphenidat, Midazolam Maleate, Mirodenafil Hydrochlorid, Montelukast, insbesondere in Form von Montelukast Natrium, Multimeric-001, Naloxon, Nikotin, Nitroglycerin, Olanzapin, Olopatadin Hydrochlorid, Ondansetron, insbesondere in Form von Ondansetron Hydrochlorid, Oxybutynin, Pektin, Pektin/Menthol, Pectin/Ascorbinsäure, PediaSUNAT (Artesunat und Amodiaquin), Piroxicam, Phenylephrin, insbesondere in Form von Phenylephrine Hydrobromid oder Hydrochlorid, Prednisolon, Pseudoephedrin, Risperidon, Rivastigmin, Rizatriptan, insbesondere in Form Rizatriptan Benzoate, Selegiline, Senna Glykosiden, Sildenafil Zitrat, Simethicon, SPO-1202, SPO-1201, SPO-1113, SPO-1108 SPO-111, Sumatriptan, Tadalafil, Testosteron, Triamcinolon Azetonid, Triptan, Tropicamide, Voglibose, Zolmitriptan, Zolpidem, insbesondere in Form von Zolpidem Tartrat. Weiterhin kann der pharmazeutische Wirkstoff für die Mundhygiene geeignet sein, wie Menthol. Der pharmazeutische Wirkstoff kann auch eine Mischung von unterschiedlichen Wirkstoffen sein.

Der orodispergierbare Film kann auch als Träger für einen Impfstoff dienen, wobei der pharmazeutische Wirkstoff in diesem Fall in Form eines Impfstoffs, z.B. als Rotavirenimpfstoff vorliegt.

Die Menge des pharmazeutischen Wirkstoffs im orodispergierbaren Film liegt vorzugsweise im Bereich von 0,001 bis 10 mg/cm$^2$, insbesondere 0,01 bis 10 mg/cm$^2$, weiter bevorzugt 2 bis 8 mg/cm$^2$ und meist bevorzugt 3 bis 7 mg/cm$^2$.

Der orodispergierbare Film hat, nach dem Gießen als Nassfilm, vorzugsweise eine Nassschichtdicke im Bereich von 400 $\mu$m bis 1500 $\mu$m und bevorzugt im Bereich von 800 bis 1200 $\mu$m. Als besonders geeignete Schichtdicke des Trockenfilms kann ein Bereich von 150 $\mu$m bis 600 $\mu$m und insbesondere 290 bis 350 $\mu$m angegeben werden.

Der Film weist weiterhin zweckmäßig eine theoretische Porosität im Bereich von 0,4 bis 0,7, insbesondere 0,52 bis 0,7, bevorzugt im Bereich von 0,55 bis 0,68 und besonders bevorzugt im Bereich von 0,58 bis 0,66 auf. Die theoretische Porosität $\varepsilon$ berechnet sich in diesem Fall gemäß der Gleichung

$$\varepsilon = \frac{V_{SOFT} - \left(\frac{m_{SOFT}}{\rho_{SOFT}}\right)}{V_{SOFT}}$$

wobei $V_{SOFT}$ das Volumen des Films, $m_{SOFT}$ die Masse

des Films und $\rho_{SOFT}$ die mittlere Dichte des Films angibt. Wie vorstehend erläutert, ist die Porosität über den Querschnitt des erfindungsgemäßen Films nicht einheitlich. Die hier angegebene Porosität stellt daher der Mittelwert der Porosität über den Querschnitt des Films dar.

Das Trocknen im Rahmen des Schritts iii) oder im Kontext der im Vorstehenden geschilderten Schritte iv) und v) erfolgt zweckmäßig in einem für das Verdampfen des jeweils verwendeten Lösungsmittels geeigneten Temperaturbereich. Die Temperatur des Trocknens ist dabei zweckmäßig zumindest im Anfangsstadium des Trocknens so gewählt, dass sie im Bereich oder leicht unterhalb der Siedetemperatur des pharmazeutisch akzeptablen Lösungsmittels liegt, um einerseits ein möglichst schnelles Verdampfen des Lösungsmittels zu erzielen, andererseits aber ein Kochen des Lösungsmittels, bei dem Blasen, die die Einheitlichkeit des Films beeinträchtigen können, entstehen, zu vermeiden. Für die meisten der im Vorstehenden erwähnten geeigneten pharmazeutisch akzeptablen Lösungsmittel kann ein Temperaturbereich von 40 bis 80°C, bevorzugt 45 bis 70°C und besonders bevorzugt 48 bis 60°C als geeignet angegeben werden.

Im Rahmen des im Vorstehenden geschilderten Verfahrens zur Herstellung eines orodispergierbaren Films ist es möglich, schon während der Bildung der Suspension im Schritt i) einen pharmazeutischen Wirkstoff zuzugeben, der dann im Rahmen des Gießens und Trocknens in den Schritten ii) und iii) direkt in den Film einbezogen wird. In diesem Fall ist es über den im Vorstehenden erläuterten Schritt iv) möglich, zusätzlich einen weiteren Wirkstoff, der von dem in dem Schritt i) verwendeten Wirkstoff verschieden sein kann, in den porösen orodispergierbaren Film einzubringen. Dadurch kann beispielsweise bei Verwendung des gleichen Wirkstoffs ausgehend von einer Basisbeladung die Menge des Wirkstoffs individuell auf die Bedürfnisse des Patienten abgestimmt werden. Falls der im Schritt iv) verwendete Wirkstoff von dem im Schritt i) verwendeten Wirkstoff verschieden ist, besteht ebenfalls Flexibilität in Bezug auf diesen zweiten Wirkstoff, die zur Anpassung der Wirkstoffmenge auf den jeweiligen Patienten genutzt werden kann.

Zur Stabilisierung des pharmazeutischen Wirkstoffs in Schritt iv) kann es zweckmäßig sein, wenn der Suspension oder Lösung des Wirkstoffs in einem pharmazeutisch akzeptablen Lösungsmittel zusätzlich ein Stabilisator für den Wirkstoff zugegeben wird. Bei diesem Stabilisator handelt es sich in einer Ausführungsform des vorstehend geschilderten Verfahrens um das in Schritt i) verwendete pharmazeutisch akzeptable Bindemittel. Ein besonders geeignetes Material für den Stabilisator ist demzufolge Hydroxypropylcellulose. Alternativ oder zusätzlich können synthetische polymere Stabilisatoren verwendet werden. Geeignete synthetische polymere Stabilisatoren sind beispielsweise Vinylpyrrolidon-Polymere und Copolymere, insbesondere Vinylpyrrolidon-Vinylacetat-Copolymere, wie sie beispielsweise unter dem Handelsnamen Kollidon® VA 64 von BASF, DE vertrieben werden.

Neben den im Vorstehenden erläuterten Bestandteilen können für die Bildung der Suspension in Schritt i), aber auch im Rahmen des Aufbringens einer Suspension oder Lösung eines pharmazeutischen Wirkstoffs im Schritt iv), weitere Inhaltsstoffe zugegeben werden. Beispiele für solche Inhaltsstoffe sind beispielsweise hydrophilisierende Mittel, wie Polyoxyethylenlaurylether, beispielsweise kommerziell erhältlich als Brij35 (zum Beispiel Merck KGaA, Deutschland), Geschmackstoffe, Geruchsstoffe, aromatische Mittel und/oder Farbmittel, wie Farbstoffe und/oder Pigmente.

Ein weiterer Aspekt der vorstehend geschilderten Erfindung betrifft einen orodispergierbaren Trockenfilm, der nach einem Verfahren, wie im Vorstehenden beschrieben, erhältlich ist. Dieser Trockenfilm zeichnet sich in Bezug auf seine Oberflächenbeschaffenheit dadurch aus, dass er auf einer Seite porös ist, was das Einbringen einer Suspension oder Lösung erlaubt, während auf der anderen Seite des Films eine geschlossene Oberfläche vorliegt, auf die eine solche Suspension oder Lösung nur aufgebracht werden kann, aber nicht in das Filmmaterial eindringt. Diese Struktur des Films wird durch das vorstehend geschilderte Verfahren erzielt.

Der Film enthält vorzugsweise ab einem Abstand von 20 $\mu$m von der Unterseite, bevorzugt ab einem Abstand von 10 $\mu$m von der Unterseite Poren, die zumindest anteilsmäßig mit nachweisbaren Mengen an pharmazeutischem Wirkstoff gefüllt sind.

Entsprechende orodispergierbare Filme können insbesondere als Medikament verwendet werden.

Für bevorzugte Ausgestaltungsformen der letztgenannten Aspekte kann auf die vorstehenden Ausführungen verwiesen werden.

Der erfindungsgemäße orodispergierbare Trockenfilm, der im Rahmen des erfindungsgemäßen Verfahrens nach Schritt iii) erzeugt wird, weist zweckmäßig eine Trockenschichtdicke im Bereich von 100 bis 600 $\mu$m, eine theoretischen Porosität im Bereich von 0,4 bis 0,7 und einer Zugfestigkeit im Bereich von 0,4 bis 4 Nmm$^{-2}$ auf. Für bevorzugte Ausgestalltungen der Trockenschichtdicke und der theoretischen Porosität, sowie des Bindemittels und des Matrixmaterials kann auf die vorstehenden Ausführungen verwiesen werden. Derartige Filme haben eine Struktur in der Gestalt, dass sie auf einer Seite des Films eine geschlossene Oberfläche aufweisen, und auf der anderen Seite porös sind, so dass eine Flüssigkeit, die auf die geschlossene Oberfläche aufgebracht wird, auf dieser verbleibt, während eine Flüssigkeit, die auf die poröse Seite des Films aufgebracht wird, in den Film eindringen kann. Darüber hinaus hat dieser orodispergierbare Trockenfilm vorzugsweise eine Desintegrationszeit im Bereich von 5 bis 250 Sekunden, wenn diese wie im Beispielteil beschrieben bestimmt wird.

Als bevorzugte Desintegrationszeit für diesen Film kann ein Zeitraum von 10 bis 120 Sekunden, und insbesondere 20 bis 60 Sekunden angegeben werden. Alternativ oder zusätzlich ist eine Zugfestigkeit von 0,6 bis 2,5 Nmm$^{-2}$ bevorzugt, und eine Zugfestigkeit von 0,8 bis 1,8 Nmm$^{-2}$ besonders bevorzugt.

Die Poren des Films in der erfindungsgemäßen Ausgestaltung sind zumindest anteilsmäßig mit einem pharmazeutischen Wirkstoff gefüllt, wodurch sich eine theoretische Porosität ergeben kann, die unterhalb des vorstehend genannten Werts liegt.

Im Folgenden wird die vorliegende Erfindung anhand einiger illustrierender Beispiele veranschaulicht, die jedoch nicht als in irgendeiner Weise beschränkend für den Schutzumfang der Anmeldung anzusehen sind.

Beispiele:

Beispiel 1

Den folgenden Untersuchungen wurden Suspensionen mit verschiedenen Matrix/Bindemittel-Verhältnissen zugrunde gelegt. Die hierzu verwendeten Suspensionen enthielten Ethanol als Lösungsmittel, HMPC (Pharmacoat 606) als Matrixmaterial und HPC (Sigma Aldrich) als Bindemittel. Die Suspension wurde durch Lösen von HPC in Ethanol und anschließende Zugabe des Matrixmaterials hergestellt. Die so erhaltene Suspension wurde für 16 Stunden entgast und anschließend mit einem automatischen Filmziehgerät ZAA 2300 (Zehntner, Schweiz) auf einen PET-Film aufgebracht. Die Ziehgeschwindigkeit betrug 10 mm s$^{-1}$ und der Abstand zum Substrat betrug 1000 $\mu$m. Die Filme wurden bei 50°C für 30 min im Ofen getrocknet und anschließend von Substrat abgelöst.

In den für die Herstellung der Filme verwendeten Suspensionen betrug der Ethanolgehalt $c_{EtOH}$ = 0,735. Der Anteil des Bindemittels, bezogen auf die Gesamtmenge an Matrixmaterial und Bindemittel ($c_{HPC}$) wurde im Bereich von 0,25 bis 0,33 variiert. Von den so erhaltenen Filmen wurde jeweils die Dicke des Films, die Desintegrationszeit und die Festigkeit des Films anhand der folgenden Methoden bestimmt.

Dicke: Die Trockenschichtdicke wurde mit einer digitalen Messuhr mit einer kreisrunden Auflagefläche auf der Probe (Durchmesser ca. 3 mm) mit einer Genauigkeit von 0,001 mm der Mitutoya Deutschland GmbH als Mittelwert von 10 unabhängigen Messungen bestimmt.

Desintegrationszeit: Die Desintegrationszeit wurde mit der SFaB (slide frame and ball) Methode bestimmt, die von Steiner in International Journal of Pharmaceutics, vol. 511, 2016, S. 804-813 beschrieben wird. Hierzu wurden Filme mit den Dimensionen 3 x 4 cm so in einen Rahmen gespannt, dass die Offenen Poren auf der Oberseite des Geräts waren. Die Messung wurde gestartet, wenn der Ball (rostfreier Stahl, $m_{Ball}$ = 4 g, d = 10 mm) auf dem ersten Tropfen aus 0,9 ml destilliertem Wasser (T = 37°C) platziert worden war. Nach dem Start der Messung wurde der Rest des Wassers auf die Filmoberfläche gegeben. Die Desintegrationszeit wurde definiert als die Zeit, die das Wasser benötigt um den orodispergierbaren Film zu zersetzen, bis der Ball durch den Film auf den Boden des Geräts 200 mm darunter fällt. Zur besseren Vergleichbarkeit wurde die spezifische Desintegrationszeit als Quotient der gemessenen Desintegrationszeit und der Filmdicke berechnet.

Zugfestigkeit: Die Zugfestigkeit wurde mit einer Materialprüfmaschine (8136/20N, Zwick GmbH & Co. KG) mit Teststreifen der Dimension 5 x 35 mm bestimmt. Die Teststreifen wurden zwischen die zwei Greifer gespannt und mit einer Geschwindigkeit von 5 mm/min auseinander gezogen. Die Maximalkraft, $F_{max}$, bis zum reißen des orodispergierbaren Films wurde aufgezeichnet und als mechanische Festigkeit des Films angegeben. Die Zugfestigkeit des Films ergibt sich als Quotient von $F_{max}$ und der Querschnittsfläche.

Die Ergebnisse der Dickebestimmungen sind in der folgenden Tabelle 1 dargestellt. Die Ergebnisse der Desintegrationszeit und die Zugfestigkeit sind in Figur 2 dargestellt.

Tabelle 1

| $c_{HPC}$ | Dicke des Films |
|---|---|
| 0,28 | 410 $\mu$m |
| 0,31 | 280 $\mu$m |
| 0,33 | 210 $\mu$m |

Es zeigt sich, dass die Dicke mit zunehmender Bindemittelkonzentration abnimmt, so dass die Filme insgesamt eine geringere Porosität aufweisen. Dies vermindert die Aufnahmekapazität für einen Wirkstoff. Andererseits wurde beobachtet, dass bei geringen Bindemittelgehalten nicht mehr sichergestellt war, dass eine auf der Unterseite geschlossene Oberfläche erhalten wurde.

Aus Figur 2 lässt sich zudem entnehmen, dass die Zugfestigkeit der Filme, aber auch deren Desintegrationszeit mit zunehmendem Bindemittelgehalten zunahm.

Beispiel 2: Beladung von orodispergierbaren Filmen mit einem Modelwirkstoff

Um die Verteilung eines Modelwirkstoffs in den erfindungsgemäßen orodispergierbaren Filmen bestimmen zu können, wurde die Beladung der Filme im Hinblick auf die eindeutige Detektion mit einer Aluminiumoxidsuspension untersucht ($x_{50}$ = 100 nm, Lösungsmittel = Ethanol). Die Verteilung des Aluminiumoxids im Film wurde dann mit SEM/EDX (Element tracking) bestimmt (sh. Figur 3). Mit diesen Untersuchungen konnte gezeigt werden, dass die Einlagerung der Partikel in die Filmporen möglich war und dass die Suspensionen den orodispergierbaren Film bis zur geschlossenen Unterseite durchdringt. Es konnte weiterhin gezeigt werden, dass sich der poröse Matrixfilm währende des Beladens nicht auflöste.

Beispiel 3: Variation der Beschichtungszyklen und Partikelkonzentration

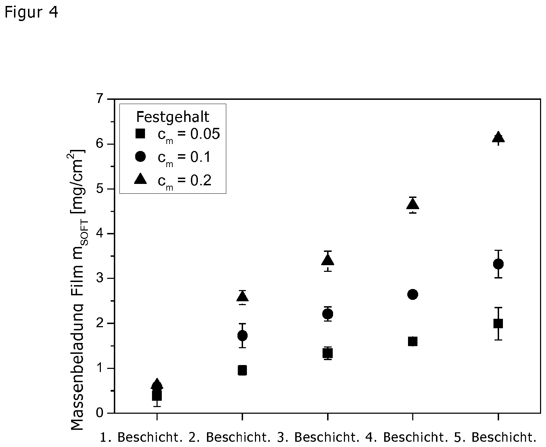

Für diese Untersuchungen wurde eine Anthrachinon-Suspension ($x_{50}$ = 400 nm) verwendet. Diese enthielt neben dem Anthrachinon HPC als Stabilisierungsmittel in einer Konzentration von $c_{HPC}$ = 0,25 (bezogen auf die Gesamtmenge an Anthrachinon in der Suspension). Die Konzentration der Suspension wurde im Bereich von 0,05 bis 0,2 variiert, und die Suspension wurde einfach bis zu fünffach auf den Film aufgetragen. Der Auftrag erfolgte über eine Applikationsdüse mit einem Volumen von 25 $\mu$m/min mit einer Geschwindigkeit von 220 mm/min auf die Filmoberfläche. Bei dem für die Untersuchungen verwendeten Film handelte es sich um einen Film mit $c_{HPC}$ = 0,28 wie er in Beispiel 1 geschildet ist.
Für die erhaltenen Filme wurde jeweils die Beladung mit Anthrachinon bestimmt. Die Ergebnisse dieser Untersuchungen sind in Figur 4 dargestellt.
In den Untersuchungen zeigte sich, dass die höchste Beladung von 6,1 mg/cm$^2$ mit einer Anthrachinonkonzentration von 0,2 in fünf Beschichtungszyklen realisiert werden konnte. Dies entspricht einer Anthrachinonbeladung von 4,9 mg/cm$^2$. Unter Berücksichtigung der Standardgröße eines orodispergierbaren Films von 6 cm$^2$ ergibt sich damit eine maximale Wirkstoffbeladung von etwa 30 mg.

**Patentansprüche**

1. Verfahren zur Herstellung eines orodispergierbaren Films, umfassend

   i) Bilden einer Suspension aus einem pharmazeutisch akzeptablen Lösungsmittel, einem pharmazeutisch akzeptablem Matrixmaterial und einem pharmazeutisch akzeptablem Bindemittel, wobei das Lösungsmittel so ausgewählt ist, dass sich das pharmazeutisch akzeptable Matrixmaterial darin im Wesentlichen nicht löst, so dass die Löslichkeit des pharmazeutisch akzeptablen Matrixmaterials in dem Lösungsmittel nicht mehr als 1 g/L bei 23°C beträgt, während das pharmazeutisch akzeptable Bindemittel im Lösungsmittel gelöst wird,
   ii) Gießen der Suspension auf einen neutralen Träger unter Bildung eines Nassfilms,
   iii) Trocknen des Nassfilms unter Erhalt eines porösen Trockenfilms, wobei das Verfahren weiterhin einen Schritt
   iv) des Aufbringens einer Suspension oder Lösung eines pharmazeutischen Wirkstoffs in einem pharmazeutisch akzeptablen Lösungsmittel auf den aus Schritt iii) erhaltenen Trockenfilm und Trocknen des Films unterzogen wird, wobei ein orodispergierbarer Trockenfilm erhalten wird, dessen Poren zumindest anteilsmäßig mit dem pharmazeutischen Wirkstoff gefüllt sind,

aufweist.

2. Verfahren gemäß Anspruch 1, **dadurch gekennzeichnet**, im Rahmen des Schritts iii) Wärme von unten über den neutralen Träger zugeführt wird.

3. Verfahren gemäß Anspruch 1, **dadurch gekennzeichnet**, im Rahmen des Schritts iii) Wärme von oben zugeführt wird.

4. Verfahren gemäß Anspruch 1, **dadurch gekennzeichnet, dass** der aus Schritt iv) erhaltene Film einem Schritt v) des Aufbringens einer Suspension oder Lösung eines pharmazeutisch akzeptablen Bindemittels in einem pharmazeutisch akzeptablen Lösungsmittel auf den aus Schritt iv) erhaltenen Trockenfilm unterzogen wird, wobei ein orodispergierbarer Trockenfilm erhalten wird, dessen Poren zumindest anteilsmäßig mit dem pharmazeutischen Wirkstoff gefüllt sind und der mit einer Schutzschicht aus dem Bindemittel überzogen ist.

5. Verfahren gemäß einem der Ansprüche 1 bis 4, **dadurch gekennzeichnet, dass** als pharmazeutisch akzeptables Lösungsmittel ein Alkohol, bevorzugt in Form von Ethanol, ein Ester oder Mischungen davon verwendet wird.

6. Verfahren gemäß einem der vorstehenden Ansprüche, **dadurch gekennzeichnet, dass** als pharmazeutisch akzeptables Matrixmaterial ein Di-Oligo- oder Polysaccharid, insbesondere in Form von Cellulose oder einem Cellulosederivat und bevorzugt in Form von Hydroxypropylmethylcellulose, verwendet wird.

7. Verfahren gemäß einem der vorstehenden Ansprüche, **dadurch gekennzeichnet, dass** das pharmazeutisch akzeptable Matrixmaterial eine mittlere Teilchengröße im Bereich von 10 bis 200 $\mu$m, insbesondere 50 bis 150 $\mu$m, bevorzugt 70 bis 130 $\mu$m und besonders bevorzugt im Bereich von 80 bis 120 $\mu$m aufweist.

8. Verfahren gemäß einem der vorstehenden Ansprüche, **dadurch gekennzeichnet, dass** als pharmazeutisch akzeptables Bindemittel ein synthetisches Polymer, bevorzugt in Form von Polyvinylpyrrolidon, oder ein Polysaccharid, bevorzugt in Form eines Cellulosederivats, besonders bevorzugt in Form von Hydroxypropylcellulose, verwendet wird.

**9.** Verfahren gemäß einem der vorstehenden Ansprüche, **dadurch gekennzeichnet, dass** der Anteil von pharmazeutisch akzeptablem Bindemittel an der Gesamtmenge an pharmazeutisch akzeptablem Matrixmaterial und Bindemittel im Bereich von 0,1 bis 0,5, insbesondere 0,2 bis 0,4, bevorzugt von 0,25 bis 0,33 und besonders bevorzugt von 0,27 bis 0,31 liegt.

**10.** Verfahren gemäß einem der vorstehenden Ansprüche, **dadurch gekennzeichnet, dass** der Anteil des Lösungsmittels, bezogen auf das Gesamtgewicht der Suspension im Bereich von 0,4 bis 0,9, insbesondere 0,68 bis 0,8, bevorzugt von 0,70 bis 0,76 und besonders bevorzugt von 0,71 bis 0,75 liegt.

**11.** Verfahren gemäß einem der vorstehenden Ansprüche, **dadurch gekennzeichnet, dass** der Film eine Nassschichtdicke im Bereich von 400 $\mu$m bis 1500 $\mu$m, bevorzugt im Bereich von 800 $\mu$m bis 1200 $\mu$m und/oder eine Trockenschichtdicke im Bereich von 150 $\mu$m bis 600 $\mu$m, bevorzugt im Bereich von 290 bis 350 $\mu$m aufweist.

**12.** Verfahren gemäß einem der vorstehenden Ansprüche, **dadurch gekennzeichnet, dass** der in Schritt iii) erhaltene Film eine theoretische Porosität $\varepsilon$ im Bereich von 0,4 bis 0,7, insbesondere 0,52 bis 0,7, bevorzugt im Bereich von 0,55 bis 0,68 und besonders bevorzugt im Bereich von 0,58 bis 0,66 aufweist.

**13.** Verfahren gemäß einem der vorstehenden Ansprüche, **dadurch gekennzeichnet, dass** das Trocknen bei einer Temperatur im Bereich von 40°C bis 80°C, bevorzugt 45°C bis 70°C und besonders bevorzugt 48°C bis 60°C durchgeführt wird.

**14.** Verfahren gemäß Anspruch 1 oder einem davon abhängigen Anspruch, **dadurch gekennzeichnet, dass** die Suspension oder Lösung eines pharmazeutischen Wirkstoffs in einem pharmazeutisch akzeptablen Lösungsmittel zusätzlich einen Stabilisator für den Wirkstoff enthält, wobei es sich bevorzugt um das in Schritt i) verwendete pharmazeutisch akzeptable Bindemittel handelt.

**15.** Verfahren gemäß Anspruch 1 oder einem davon abhängigen Anspruch, **dadurch gekennzeichnet, dass** der Film nach dem Trocknen eine Wirkstoffbeladung im Bereich von 0,01 bis 10 mg/cm$^2$, bevorzugt 2 bis 8 mg/cm$^2$ und besonders bevorzugt 3 bis 7 mg/cm$^2$ aufweist.

**16.** Orodispergierbarer Trockenfilm, erhältlich nach einem Verfahren gemäß einem der Ansprüche 1 bis 15.

**Claims**

**1.** A method for producing an orodispersible film, comprising the following steps:

i) forming a suspension of a pharmaceutically acceptable solvent, a pharmaceutically acceptable matrix material, and a pharmaceutically acceptable binder, said solvent being selected such that the pharmaceutically acceptable matrix material substantially does not dissolve in it, so that the solubility of the pharmaceutically acceptable matrix material in the solvent is not more than 1 g/L at 23°C, whereas the pharmaceutically acceptable binder is dissolved in the solvent,
ii) casting the suspension onto a neutral support, thereby forming a wet film, and
iii) drying the wet film and obtaining a dry porous film, wherein the method further comprises a step of
iv) applying a suspension or solution of a pharmaceutically active ingredient in a pharmaceutically acceptable solvent to the dry film obtained from step iii) and drying the film, an orodispersible dry film being obtained, the pores of which are filled at least is part with the pharmaceutical active ingredient.

**2.** The method according to claim 1, **characterised in that** within the scope of step iii) heat is fed from below via the neutral support.

**3.** The method according to claim 1, **characterised in that** within the scope of step iii) heat is fed from above.

**4.** The method according to claim 1, **characterised in that** the film obtained from step iv) is subjected to a step v) of applying a suspension or solution of a pharmaceutically acceptable binder in a pharmaceutically acceptable solvent to the dry film obtained from step iv), an orodispersible dry film being obtained, the pores of which are filled at least in part with the pharmaceutical active ingredient and which is coated by a protective layer of the binder.

**5.** The method according to any one of claims 1 to 4, **characterised in that** an alcohol, preferably in the form of ethanol, an ester or mixtures thereof, is used as pharmaceutically acceptable solvent.

**6.** The method according to any one of the preceding claims, **characterised in that** a di-, oligo or polysaccharide, especially in the form of cellulose or a cellulose derivative and preferably in the form of hydroxypropyl methyl cellulose, is used as pharmaceutically acceptable matrix material.

**7.** The method according to any one of the preceding claims, **characterised in that** the pharmaceutically acceptable matrix material has a mean particle size in the range of from 10 to 200 $\mu$m, especially 50 to 150 $\mu$m, preferably 70 to 130 $\mu$m, and especially preferably in the range of from 80 to 120 $\mu$m.

**8.** The method according to any one of the preceding claims, **characterised in that** a synthetic polymer, preferably in the form of polyvinyl pyrrolidone, or a polysaccharide, preferably in the form of a cellulose derivative, especially preferably in the form of hydroxypropyl cellulose, is used as pharmaceutically acceptable binder.

**9.** The method according to any one of the preceding claims, **characterised in that** the proportion of pharmaceutically acceptable binder in the total quantity of pharmaceutically acceptable matrix material and binder is in the range of from 0.1 to 0.5, especially 0.2 to 0.4, preferably from 0.25 to 0.33, and especially preferably from 0.27 to 0.31.

**10.** The method according to any one of the preceding claims, **characterised in that** the proportion of solvent, in relation to the total weight of the suspension, is in the range of from 0.4 to 0.9, especially 0.68 to 0.8, preferably from 0.70 to 0.76, and especially preferably from 0.71 to 0.75.

**11.** The method according to any one of the preceding claims, **characterised in that** the film has a wet-layer thickness in the range of from 400 $\mu$m to 1500 $\mu$m, preferably in the range of from 800 $\mu$m to 1200 $\mu$m, and/or a dry-layer thickness in the range of from 150 $\mu$m to 600 $\mu$m, preferably in the range of from 290 to 350 $\mu$m.

**12.** The method according to any one of the preceding claims, **characterised in that** the film has a theoretical porosity $\varepsilon$ in the range of from 0.4 to 0.7, especially 0.52 to 0.7, preferably in the range of from 0.55 to 0.68, and especially preferably in the range of from 0.58 to 0.66.

**13.** The method according to any one of the preceding claims, **characterised in that** the drying is performed at a temperature in the range of from 40°C to 80°C, preferably 45°C to 70°C and especially preferably 48°C to 60°C.

**14.** The method according to claim 1, or a claim dependent thereon, **characterised in that** the suspension or solution of a pharmaceutical active ingredient in a pharmaceutically acceptable solvent additionally contains a stabiliser for the active ingredient, this preferably being the pharmaceutically acceptable binder used in step i).

**15.** The method according to claim 1, or a claim dependent thereon, **characterised in that** the film after drying has an active ingredient loading in the range of from 0.01 to 10 mg/cm$^2$, preferably 2 to 8 mg/cm$^2$, and especially preferably 3 to 7 mg/cm$^2$.

**16.** An orodispersible dry film obtainable by a method according to any one of claims 1 to 15.

**Revendications**

**1.** Procédé de fabrication d'un film orodispersible, comprenant

i) la formation d'une suspension à partir d'un solvant pharmaceutiquement acceptable, d'un matériau de matrice pharmaceutiquement acceptable et d'un liant pharmaceutiquement acceptable, dans lequel le solvant est choisi de telle sorte que le matériau de matrice pharmaceutiquement acceptable ne s'y dissout pas sensiblement si bien que la solubilité du matériau de matrice pharmaceutiquement acceptable dans le solvant ne dépasse pas 1 g/l à 23 °C, tandis que le liant pharmaceutiquement acceptable est dissous dans le solvant,
ii) le déversement de la suspension sur un support neutre avec formation d'un film humide,
iii) le séchage du film humide avec obtention d'un film sec poreux, dans lequel le procédé présente par ailleurs une étape
iv) d'application d'une suspension ou d'une solution d'un principe actif pharmaceutique dans un solvant pharmaceutiquement acceptable sur le film sec obtenu à l'étape iii) et de séchage du film, dans lequel le film sec orodispersible est obtenu, dont les pores sont remplis au moins proportionnellement du principe actif pharmaceutique.

**2.** Procédé selon la revendication 1, **caractérisé en ce que** de la chaleur est amenée depuis le bas au-dessus du support neutre dans le cadre de l'étape iii).

**3.** Procédé selon la revendication 1, **caractérisé en ce que** de la chaleur est amenée depuis le bas dans le cadre de l'étape iii).

**4.** Procédé selon la revendication 1, **caractérisé en ce que** le film obtenu à l'étape iv) est exposé à une étape v) d'application d'une suspension ou d'une solution d'un liant pharmaceutiquement acceptable dans un solvant pharmaceutiquement acceptable sur le film sec obtenu à l'étape iv), dans lequel un film sec orodispersible est obtenu, dont les pores sont remplis au moins proportionnellement du principe actif pharmaceutique, et est recouvert d'une couche de protection composée du liant.

5. Procédé selon l'une quelconque des revendications 1 à 4, **caractérisé en ce qu'**un alcool, de manière préférée sous la forme d'éthanol, d'un ester ou d'un mélange de ceux-ci, est utilisé en tant que solvant pharmaceutiquement acceptable.

6. Procédé selon l'une quelconque des revendications précédentes, **caractérisé en ce qu'**un di-oligosaccharide ou polysaccharide, en particulier sous la forme de cellulose ou d'un dérivé de cellulose et de manière préférée sous la forme d'hydroxypropylméthylcellulose, est utilisé en tant que matériau de matrice pharmaceutiquement acceptable.

7. Procédé selon l'une quelconque des revendications précédentes, **caractérisé en ce que** le matériau de matrice pharmaceutiquement acceptable présente une taille de particule moyenne dans la plage de 10 à 200 $\mu$m, en particulier de 50 à 150 $\mu$m, de manière préférée de 70 à 130 $\mu$m et de manière particulièrement préférée dans la plage de 80 à 120 $\mu$m.

8. Procédé selon l'une quelconque des revendications précédentes, **caractérisé en ce qu'**un polymère synthétique, de manière préférée sous la forme de polyvinylpyrrolidone, ou un polysaccharide, de manière préférée sous la forme d'un dérivé de cellulose, de manière particulièrement préférée sous la forme d'hydroxypropylcellulose, est utilisé en tant que liant pharmaceutiquement acceptable.

9. Procédé selon l'une quelconque des revendications précédentes, **caractérisé en ce que** la proportion de liant pharmaceutiquement acceptable sur la quantité totale de matériau de matrice pharmaceutiquement acceptable et de liant se situe dans la plage de 0,1 à 0,5, en particulier de 0,2 à 0,4, de manière préférée de 0,25 à 0,33, et de manière particulièrement préférée de 0,27 à 0,31.

10. Procédé selon l'une quelconque des revendications précédentes, **caractérisé en ce que** la proportion du solvant par rapport au poids total de la suspension se situe dans la plage de 0,4 à 0,9, en particulier de 0,68 à 0,8, de manière préférée de 0,70 à 0,76 et de manière particulièrement préférée de 0,71 à 0,75.

11. Procédé selon l'une quelconque des revendications précédentes, **caractérisé en ce que** le film présente une épaisseur de couche humide dans la plage de 400 $\mu$m à 1500 $\mu$m, de manière préférée dans la plage de 800 $\mu$m à 1200 $\mu$m et/ou une épaisseur de couche sèche dans la plage de 150 $\mu$m à 600 $\mu$m, de manière préférée dans la plage de 290 à 350 $\mu$m.

12. Procédé selon l'une quelconque des revendications précédentes, **caractérisé en ce que** le film obtenu à l'étape iii) présente une porosité théorique $\varepsilon$ dans la plage de 0,4 à 0,7, en particulier de 0,52 à 0,7, de manière préférée dans la plage de 0,55 à 0,68 et de manière particulièrement préférée dans la plage de 0,58 à 0,66.

13. Procédé selon l'une quelconque des revendications précédentes, **caractérisé en ce que** le séchage est effectué à une température dans la plage de 40 °C à 80 °C, de manière préférée de 45 °C à 70 °C et de manière particulièrement préférée de 48 °C à 60 °C.

14. Procédé selon la revendication 1 ou une revendication dépendante de celle-ci, **caractérisé en ce que** la suspension ou la solution d'un principe actif pharmaceutique dans un solvant pharmaceutiquement acceptable contient en supplément un agent stabilisant pour le principe actif, dans lequel il s'agit de manière préférée du liant pharmaceutiquement acceptable utilisé lors de l'étape i).

15. Procédé selon la revendication 1 ou une revendication dépendante de celle-ci, **caractérisé en ce que** le film présente après le séchage une charge en principe actif dans la plage de 0,01 à 10 mg/cm$^2$, de manière préférée de 2 à 8 mg/cm$^2$ et de manière particulièrement préférée de 3 à 7 mg/cm$^2$.

16. Film sec orodispersible pouvant être obtenu selon un procédé selon l'une quelconque des revendications 1 à 15.

Figur 1

Oberseite

Unterseite

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007009800 A
- WO 2007009801 A
- WO 03011259 A
- WO 2013023775 A1

**In der Beschreibung aufgeführte Nicht-Patentliteratur**

- **VON STEINER.** *International Journal of Pharmaceutics,* 2016, vol. 511, 804-813